# EUROPÄISCHE PATENTSCHRIFT

(11) **EP 1 660 179 B1**
(45) Veröffentlichungstag und Bekanntmachung des Hinweises auf die Patenterteilung: **03.01.2007**
(21) Anmeldenummer: 04762755.9
(22) Anmeldetag: 03.09.2004
(51) Int. Cl.: A61N 1/362

(54) **VORRICHTUNG ZUR MUSKELSTIMULATION**
DEVICE FOR MUSCLE STIMULATION
DISPOSITIF DE STIMULATION MUSCULAIRE

(30) Priorität: 03.09.2003 DE 10341044; 24.02.2004 DE 102004009452
(43) Veröffentlichungstag der Anmeldung: 31.05.2006
(73) Patentinhaber: Fides Finanz-Invest Gmbh & CO.KG, 23669 Timmendorfer Strand (DE)
(72) Erfinder: KLAPPROTH, Peter, 23562 Lübeck (DE); ULBRICH, Eckart, D-23669 Timmendorfer Strand (DE)
(74) Vertreter: Griepenstroh, Jörg
(86) Internationale Anmeldenummer: PCT/DE2004/001970
(87) Internationale Veröffentlichungsnummer: WO 2005/023363

(56) Entgegenhaltungen:
- US-A- 4 773 401
- US-A- 5 016 632
- US-A- 5 271 396
- US-A- 5 292 341

## Beschreibung

Die Erfindung betrifft eine Vorrichtung zur Muskelstimulation mit den Merkmalen des Patentanspruchs 1.

Muskulär angetriebene Herzunterstützungssysteme (z.B. Kardio-Myoplastik, Aorto-Myoplastik, Skelettmuskelventrikel, biomechanische Herzen) werden zum Teil bereits klinisch, zum Teil noch experimentell als ergänzende oder ersetzende Therapie der Herz-Transplantation und Behandlung einer terminalen Herzinsuffizienz eingesetzt. Diese muskulären Unterstützungssysteme können dabei sowohl parallel als auch seriell zum erkrankten Herzen arbeiten. Sie dienen zum einen zu dessen Entlastung (Reduktion der kardialen Wandspannung, Druckentlastung, Volumenentlastung, Nachlastsenkung) und zum anderen zur Unterstützung des Blutkreislaufs, d.h. zur Mitteldruckerhöhung des arteriellen Blutdrucks und/oder zur Steigerung des Pumpvolumens. Unabhängig von der Konfiguration des Herzunterstützungssystems wird zur gezielten Anregung einer Muskelkontraktion ein Muskelschrittmacher benötigt, der herzsynchronisiert ein elektrisches Stimulationsmuster mittels Stimulationselektroden an den unterstützenden Muskel abgibt. Ein Stimulationsmuster besteht dabei aus einer Summe von Einzelimpulsen, die sich durch ihre Stimulationsspannung, der Impulsbreite und dem Abstand zweier Impulse beschreiben lassen. Durch sinnvolle Zusammenfassung mehrerer Einzelimpulse zu einer Gruppe von Einzelimpulsen mit nachfolgender Pause entsteht ein Stimulationsburst, der für die zyklische Kontraktion und Relaxation des Muskelgewebes genutzt wird. Zur Beschreibung eines Stimulationsmusters kommt neben den vorangegangenen Parametern noch die Anzahl der Stimulationsimpulse pro Burst und die Häufigkeit der Applikation eines Stimulationsbursts hinzu. Ein weiterer Parameter ist die Platzierung des Stimulationsbursts innerhalb des Herzzyklusses, der durch eine Verzögerungszeit zu definieren ist.

An Großtieren zeigten Untersuchungen, dass eine dauerhafte und häufige Applikation von Stimulationsbursts zu einer Faserumwandlung der stimulierten Muskulatur führt, wobei die entstehenden Muskelfasern zwar weitgehend ermüdungsfrei, jedoch schwach und langsam sind. Es konnte beobachtet werden, dass bei einer dauerhaften und häufigen Applikation von Stimulationsbursts die Muskelfaserquerschnitte erheblich abnahmen. Diese hauptsächlich durch Typ-I-Muskelfasern bestimmte Muskulatur ist nur im geringen Maß geeignet, eine kreislaufwirksame Pumparbeit zu verrichten. Experimentelle Untersuchungen zeigen jedoch, dass vor einer Transformation der stimulierten Muskelfasern in Typ-I-Muskelfasern, eine Zwischenform der Transformation in schon ermüdungsfreie, aber noch kräftige Typ-IIa-Muskelfasern erreicht werden kann. Diese von Typ-IIa-Muskelfasern dominierte, schnelle und gleichzeitig kräftige Muskulatur bleibt jedoch nur erhalten, wenn die Anzahl der applizierten Stimulationsimpulse pro Zeiteinheit unterhalb eines oberen Grenzwertes bleibt. Eine Stimulation oberhalb dieser Obergrenze führt zwangsläufig zur Transformation der Typ-IIa-Muskelfasern zu Typ-I-Muskelfasern, wobei ein Verlust an Muskelkraft und Schnelligkeit einhergeht.

Bestehende Muskelschrittmachersysteme sind in der Lage vordefinierte bzw. berechnete Stimulationsbursts synchron und getriggert vom Herzrhythmus in einem einstellbaren Verhältnis Muskelkontraktion zu Herzkontraktion abzugeben. Dieses Verhältnis kann abhängig von der Herzfrequenz vordefiniert werden. Besteht z.B. über einen längeren Zeitraum eine hohe Herzfrequenz, so wird ebenfalls stets die gleiche und hohe Anzahl an Stimulationsimpulsen vom Muskelschrittmacher abgegeben. Dies führt zu einer übermäßig häufigen Benutzung des muskulären Herzunterstützungssystems mit der Konsequenz einer weitergehenden Transformation der Muskelfasern zu schwachen und langsamen Typ-I-Muskelfasern. Muskulär angetriebene Herzunterstützungssysteme verlieren dadurch ihr Unterstützungspotential, wodurch das erkrankte Herz nicht mehr effektiv entlastet werden kann. Mit weiterer Benutzung verschlechtert sich die Kreislaufunterstützung, wodurch die Herzfrequenz des Patienten kompensatorisch weiter ansteigen kann. Als Konsequenz kann es zu einer vollständigen Degeneration des unterstützten Muskels kommen.

Die DE 101 52 741 A1 offenbart ein Herztherapiegerät, insbesondere als implantierbaren Defibrillator, Herzschrittmacher oder kombinierter Herzschrittmacher-Defibrillator, mit einer Auswertungs- und Steuereinrichtung zur Auswertung einer gemessenen Herzrate, welche einen Dreibereichsspeicher zur Speicherung eines ersten, zweiten und dritten, mit aufsteigenden Werten der Herzrate aneinander angrenzenden Wertbereiches von Vergleichs-Herzraten, einen Herzratendiskriminator zur Zuordnung einer gemessenen Herzrate zu dem ersten, zweiten oder dritten Wertebereich und eine Stabilitäts-Auswertungseinrichtung zur Auswertung der Herzraten-Stabilität über einen vorbestimmten Zeitraum bei Feststellung einer im zweiten Wertebereich liegenden Herzrate aufweist. Wird eine im zweiten Wertebereich liegende Herzrate erfasst, wird eines von zwei verschiedenen Therapiesteuersignalen ausgegeben, und zwar in Abhängigkeit von der Stabilität der Herzrate. Das Stabilitätskriterium dient zur Unterscheidung zwischen "schneller" Tachykardie ("flutter") und Fibrillationen mit - noch - relativ geringer Frequenz. Gegenstand der US 4 406 287 ist ebenfalls ein Schrittmacher. Mit dieser Vorrichtung soll eine Tachykardie beendet werden, wobei unterschiedliche Impulsanzahlen/Impulsraten zum Einsatz kommen können. Wurde eine Tachykardie nach Einsatz einer ersten Kombination aus Impulsanzahl und Impulsrate nicht beendet, schließt sich eine Phase mit einer zweiten geänderten Kombination aus Impulsanzahl und lmpulsrate an. Die letzte erfolgreiche Kombination der Impulsanzahl und lmpulsrate wird im Folgenden gespeichert und als Startwert für die nächste Tachykardiebeendung verwandt. Auf diese Weise soll die Wahrscheinlichkeit der Beendigung einer Tachykardie erhöht werden.

In der US 2003/0 083 703 A1 wird ein Verfahren und eine Anordnung zur Bereitstellung eines Anti-Tachykardie-Impulsmusters offenbart. Auch mit dieser Vorrichtung ist es möglich, festzustellen, ob ein lmpulsmuster die Tachykardie beendet hat und ob eine Veränderung des Pulsmusters zweckmäßig ist.

Hiervon ausgehend liegt der Erfindung die Aufgabe zugrunde, einen Muskelstimulator bereit zu stellen, der es ermöglicht, Typ-IIa-Muskelfasern zu erhalten, um insbesondere ein leistungsfähiges und bei Bedarf arbeitendes muskuläres Herzunterstützungssystem zu schaffen. Ferner soll der Muskelstimulator die Fähigkeit besitzen, durch geeignete elektrische Stimulation eine Neubildung von Gefäßen und Kapillaren zur optimalen Blutversorgung der Muskulatur (Neovaskularisation) zu induzieren.

Diese Aufgabe kann mit einem Muskelstimulator gemäß den Merkmalen des Patentanspruchs 1 gelöst werden.

Die Unteransprüche enthalten zweckmäßige, nicht selbstverständliche Ausgestaltungen des Erfindungsgedankens.

Gegenstand des Patentanspruchs 1 ist ein Muskelstimulator, der eine Impulsgeneratoreinheit zur Erzeugung und zum Aussenden eines elektrischen Stimulationsimpulses umfasst, sowie eine Steuereinheit zum Steuern der Impulsgeneratoreinheit. Über die Steuereinheit kann die Amplitude, d.h. die Stimulationsspannung, die Frequenz, die zeitliche Verteilung der Stimulationsimpulse, die Art und Häufigkeit der Unterstützungsmodi und die Verzögerungszeit zur R-Zacke, der Tag-/Nacht-Rhythmus sowie die Phasenlage des Stimulationsimpulses eingestellt werden. Ausgehend von der Steuereinheit werden die Stimulationsimpulse über Leitungsmittel an einen oder mehrere zu stimulierende Muskel abgegeben. Des Weiteren weist der erfindungsgemäße Muskelstimulator eine Erfassungseinheit zur Erfassung des momentanen spontanen oder stimulierten Herzrhythmus des Trägers des Muskelstimulators auf. Die Erfassungseinheit dient zur Erfassung der R-Zacke, die als Grundlage für die Triggerung der Stimulationsimpulse und zur Berechnung der zeitlichen Verzögerung zwischen der R-Zacke des Herzrhythmus und dem Stimulationsburst dient. Die lmpulsgeneratoreinheit, die Steuereinheit und die Erfassungseinheit sind in einem gemeinsamen Gehäuse aufgenommen, das extrakorporal getragen werden kann oder in den Körper des Patienten implantierbar ist.

Wesentlich ist, dass zusätzlich eine Zähleinheit und eine Speichereinheit zum Zählen und zur Speicherung der Anzahl der innerhalb eines definierbaren Zeitraums abgegebenen Stimulationsimpulse vorgesehen sind. Des Weiteren ist eine Bestimmungseinheit zur Bestimmung einer mittleren Stimulationsfrequenz innerhalb des definierbaren Zeitraums vorgesehen.

Die mittlere Stimulationsfrequenz im Sinne der Erfindung ist der Quotient aus der von der Speichereinheit gespeicherten innerhalb eines definierbaren Zeitraums abgegebenen Stimulationsimpulse und dem definierten Zeitraum, in dem die Stimulationsimpulse gezählt und gespeichert worden sind (Erfassungszeitraum/Beobachtungszeitraum). Es handelt sich also um eine arithmetisch gemittelte Stimulationsfrequenz, wobei nachfolgend der Begriff mittlere Stimulationsfrequenz stellvertretend für arithmetisch gemittelte Stimulationsfrequenz verwendet wird.

Die Zähleinheit und/oder die Speichereinheit und/oder die Bestimmungseinheit sind nicht notwendigerweise innerhalb des oben genannten Gehäuses aufgenommen, sondern können in einem separaten Gehäuse, insbesondere einem extrakorporal getragenen Gehäuse untergebracht sein.

Um eine übermäßige Stimulation des stimulierten Muskels und damit eine unerwünschte Transformation der stimulierten Muskelfasern hin zu einer schwachen, langsamen und damit ineffektiven Typ-I-Muskulatur zu vermeiden, ist es erforderlich, die innerhalb eines definierbaren Zeitraums abgegebenen Stimulationsimpulse zu zählen und einer Auswertung zuzuführen. Diese Aufgabe übernimmt die Zähleinheit in Verbindung mit der Speichereinheit und einer Überwachungseinheit. Die mittlere Stimulationsfrequenz ist ein Maß für die Häufigkeit der Stimulation des Muskels in einem bestimmten Zeitraum. Zur Vermeidung von Muskelschäden darf diese mittlere Stimulationsfrequenz einen individuell zu bestimmenden Grenzwert nicht dauerhaft überschreiten.

Jeder ausgegebene Stimulationsimpuls wird gezählt und über einen längeren Betrachtungszeitraum als mittlere Stimulationsfrequenz berechnet. Ein längerer Beobachtungszeitraum im Sinne der Erfindung umfasst zumindest 30 Minuten, insbesondere aber eine oder mehrere Stunden. Zweckmäßig sind Beobachtungszeiträume von 12 oder 24 Stunden. Die mittlere Stimulationsfrequenz ist individuell bei jedem Patienten zu bestimmen und darf einen Maximalwert von 0,2 bis 2 Impulsen pro Sekunde (Hz), insbesondere 0,7 bis 1 Hz, nicht überschreiten, um eine Überbeanspruchung des Muskels und eine mittelfristige Muskelzerstörung zu vermeiden. Wichtig ist daher, die mittlere Stimulationsfrequenz hinsichtlich des gewünschten Muskeltransformations- und Erhaltungseffekt zu bewerten und in Abhängigkeit von dem Bewertungsergebnis die Stimulationsimpulsabgabe zu steuern. Hierzu ist eine kontinuierlich arbeitende Bewertungseinheit zur Einhaltung von Grenzwerten für die mittlere Stimulationsfrequenz vorgesehen, wobei die Grenzwerte in einem Bereich von 0,2 Stimulationsimpulsen pro Sekunde bis 2 Stimulationsimpulsen pro Sekunde liegend, individuell festlegbar sind. Impulseinsparmittel dienen zur Anpassung und insbesondere Reduzierung der mittleren Stimulationsfrequenz in Abhängigkeit von der erfassten mittleren Stimulationsfrequenz und den vorgegebenen Sollwerten der Bewertungseinheit. Die Impulseinsparmittel umfassen eine Berechnungseinheit zur Berechnung eines modifizierten Stimulationsmusters nach einer Gleichung, die abhängig von der mittleren Stimulationsfrequenz ist. Des Weiteren kann ein Speichermodul zur Speicherung des zeitlichen Verlaufs der Anzahl der abgegebenen Stimulationsimpulse vorgesehen sein, so wie ein Mittel zur programmgesteuerten Übertragung der mittleren Stimulationsfrequenz von der Bestimmungseinheit zur Berechnungseinheit. Des Weiteren kann eine Analyseeinheit vorgesehen sein, zur Ermittlung wann und wie oft bestimmte Grenzwerte der Herzfrequenz und/oder der mittleren Stimulationsfrequenz über- oder unterschritten wurden.

In räumlicher Hinsicht können die Zähleinheit und die Speichereinheit in dem einen Gehäuse aufgenommen sein. In das Gehäuse können zudem die Bestimmungseinheit und/oder das Impulseinsparmittel integriert sein. Optional können auch das Speichermodul und/oder die Analyseeinheit in das die Steuereinheit aufnehmende Gehäuse integriert sein. Das Gehäuse ist in den Körper des Trägers der erfindungsgemäßen Vorrichtung implantierbar. Dem Gehäuse ist zusätzlich ein Energiespeicher zugeordnet, welcher vorzugsweise transkutan aufladbar ist. Dadurch erhöht sich die Laufzeit des implantierten Teils der Vorrichtung.

Selbstverständlich ist es im Rahmen der Erfindung auch möglich, dass die Zähleinheit und/oder die Speichereinheit und/oder die Bestimmungseinheit und/oder die Impulseinsparmittel und/oder das Speichermodul und/oder die Analyseeinheit Bestandteil einer stationären und/oder vom Träger der erfindungsgemäßen Vorrichtung extrakorporal getragenen Monitoreinheit sind. An dieser Monitoreinheit sind die mittlere Stimulationsfrequenz und/oder ein Größenbereich, in welchen die mittlere Stimulationsfrequenz fällt, durch Anzeigemittel optisch und/oder akustisch und/oder haptisch darstellbar. Die Monitoreinheit besitzt optional eine Programmiereinrichtung zur Erzeugung eines Programmiersignals und eine Übertragungseinrichtung zur Übertragung des Programmiersignals an eine Sende- und Empfangseinheit in dem die Steuereinheit aufnehmenden Gehäuse. In der Monitoreinheit können zusätzlich Mittel zum Senden und Empfangen von Positionsdaten vorgesehen sein. Die Monitoreinheit umfasst optional Mittel zum Senden und Empfangen von Funksignalen zur Übertragung von patientenphysiologischen Daten an eine Anzeige- und Auswerteeinheit eines Empfängers.

Ein Gegenstand der Erfindung ist eine Kombination speziell entwickelter elektronischer Geräte zur Erzeugung und zum Erhalt von Typ-Ila-Muskelfasern für muskuläre Herzunterstützungssysteme, insbesondere für muskuläre Blutpumpen. Die Erfindung betrifft ebenfalls einen externen Myostimulator, der so programmierbar ist, dass durch eine perkutane Stimulation bereits präoperativ wenig ermüdbare Typ-IIa-Muskelfasern erzeugt werden. Es kann sich hierbei auch um eine Anordnung handeln, umfassend einen implantierbaren Myostimulator und eine Monitoreinheit, die den Muskel wirkungsvoll mit Typ-lla-Muskelfasern zur Kontraktion bringt, den Umbau in Typ-I-Muskelfasern verhindert und außerdem eine Muskelzerstörung durch Überlastung vermeidet. Die Monitoreinheit zu diesem implantierbaren Myostimulator dient als Programmiergerät sowie als Mess- und Anzeigegerät. Diese Monitoreinheit kann drahtlos oder auch drahtgebunden Informationen vom Myostimulator zum Patienten oder auch zum behandelnden Arzt übertragen. Weitere Funktionen wie eine Übertragung des Patienten-EKGs sowie ein Patienten-Ortungssystem für Notfälle können in diesem Gerät untergebracht sein. Dieses Gerät ist zudem in der Lage, für Myostimulatoren anderer Hersteller in beschränktem Umfang die zuvor benannten Funktionen zu realisieren.

Die Erfindung wird nachfolgend anhand von schematisch dargestellten Ausführungsbeispielen näher erläutert. Es zeigen:
- Figur 1: eine schematische Darstellung, wie einzelne physikalische Elemente einer erfindungsgemäßen Vorrichtung miteinander verschaltet sind;
- Figur 2: eine Darstellung der Funktionsbausteine einer Vorrichtung zur Muskelstimulation in einer ersten Ausführungsform;
- Figur 3: die Ausführungsform der Figur 1 in einer Abwandlung;
- Figur 4: eine weitere Abwandlung der Vorrichtung zur Muskelstimulation mit zusätzlichen Funktionselemente.

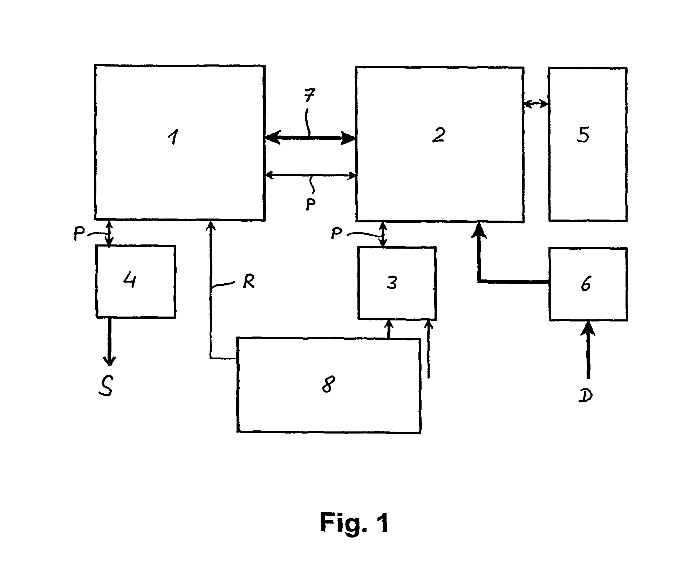

In der Ausführungsform der Figur 1 werden als programmierbare Bausteine zwei Mikrocontroller 1, 2 verwendet. Sie zeichnen sich durch eine hohe Anzahl an programmierbaren Ports, hohe Verarbeitungsgeschwindigkeit bei moderatem Stromverbrauch und Flash-Speicher mit In-System-Programmierbarkeit (ISP) aus. Weitere Bausteine des Muskelschrittmachers sind ein 12 Bit Analog-Digital-Wandler (A/D-Wandier) 3, ein 12 Bit Digital-Analog-Wandler (D/A-Wandler) 4, mehrere Operationsverstärker sowie eine Telemetrieeinheit 5. Die Stromversorgung erfolgt über langlebige Batterien, vorzugsweise über Lithiumlonen-Batterien.

Der digitale Anteil des Muskelschrittmachers besteht im wesentlich aus zwei logisch getrennten Bereichen, die jeweils mit einem eigenen Mikrocontroller 1, 2 als programmierbare Bausteine betrieben werden. Der erste Bereich, gesteuert von dem Mikrocontroller 1 dient der Erzeugung, der Verstärkung und Verteilung der Stimulationsimpulse S (max. 40 mA) auf vier Muskelelektroden und zwei kardiale Elektroden. Der zweite Bereich, gesteuert durch den Mikrocontroller 2 dient der Überwachung des Patienten, der Erfassung und Speicherung von Messdaten, z.B. von Druckdaten D, die in einer Drucklogik 6 verarbeitet und dem als Messeinheit fungierenden Mikrocontroller 2 zugeführt werden, sowie der telemetrischen Kommunikation mit der Umgebung. Über eine Mehrzweckverbindung in Form einer seriellen Schnittstelle 7 sind die beiden logischen Bereiche miteinander verbunden. Programmiert werden die beiden Mikrocontroller 1, 2 zum Beispiel in der Programmiersprache "C". Sie erhalten dabei, spezifisch ihrer Funktion, unterschiedliche Software.

Nachfolgend werden die Funktionen der zuvor genannten logisch getrennten Bereiche näher erläutert.

### Bereich 1:

### Triggerung auf die Herzaktion:

Um einen Stimulationsimpuls innerhalb des Herzzyklus geeignet zu platzieren, erhält der Mikrocontroller 1 synchron zu jeder Herzaktion ein Trigger-Signal von einer Filter-Schaltung (R-Zacken-Einheit) 8, bestehend aus 8 kaskadierten Operationsverstärkern OPV, wobei die ersten 4 OPVs das eingehende EKG-Signal mit einer Amplitude von etwa 1-10 mV auf 2-3 V verstärken und Störfrequenzen von 50 Hz und 60 Hz ausfiltern, und die weiteren 4 Stufen der Extraktion der R-Zacke und Erzeugung eines Schmitt-Trigger-Signals dienen. Dieses Trigger-Signal wird vom Mikrocontroller 1 erfasst und der zeitliche Abstand zum vorherigen Trigger-Signal bestimmt. Liegt eine Regelmäßigkeit im Vergleich zu den letzten 10 zeitlichen Trigger-Abständen vor, wird ein stabiler Sinusrhythmus des Herzen angenommen und aus dem Abstand der Trigger-Signale R die Herzfrequenz bestimmt. Die mit P bezeichneten Pfeile sind jeweils serielle Hochgeschwindigkeitsverbindungen.

### Unterstützungsmodi

In Abhängigkeit der Herzfrequenz sind verschiedene Verhältnisse einer Muskelkontraktion zu Herzkontraktion erwünscht. Der einstellbare Bereich liegt zwischen 1:1, d.h. jede Herzaktion wird durch eine Muskelaktion unterstützt und 1:255, d.h. erst nach 255 Herzaktionen erfolgt eine Muskelkontraktion. Es können bis zu 5 verschiedene Unterstützungsmodi in Abhängigkeit der Herzfrequenz definiert werden.

### Tag-Nacht-Rhythmus bzw. Arbeits-Ruhe-Rhythmus

Es können Tag- und Nachtzeiten sowie unabhängig davon Arbeits- und Ruhezeiten für den Muskelschrittmacher definiert werden, in denen der Muskelschrittmacher zwischen Impulsmustern hoher Aktivität bzw. Impulsmustern niedriger oder keiner Aktivität wechselt. Ein Wechsel zwischen Tag- und Nachtrhythmus bzw. Arbeits-Ruhe-Rhythmus kann sowohl zeitabhängig, ereignisgesteuert als auch manuell über ein patienteneigenes Steuergerät erfolgen.

### Zeitlicher Ablauf vor einer Muskelstimulation

Beim Eintreffen eines Herz-Trigger-Signals R und Nachweis der Regelmäßigkeit wird solange ein Zähler aufaddiert, bis ein zu unterstützender Herzschlag erreicht ist. Im Folgenden wird ein zweiter Zähler aktiviert, der eine bestimmte Zeit als Verzögerung bis zur Erzeugung des Stimulationsmusters verstreichen lässt. (R-Zacken-Verzögerung, R-Delay).

### Zeitlicher Ablauf während einer Muskelstimulation

Nach Ablauf der R-Zacken-Verzögerungszeit (R-Delay) wird in Abhängigkeit der Voreinstellung das Stimulationsmuster impulsweise mit den Variablen: Amplitude, Impulsbreite, Impulsphase (positiv, negativ, biphasisch) und Interimpulsabstand mit Hilfe des D/A-Wandlers 4 und einer Operationsverstärkerschaltung (OPV) generiert und auf definierte Stimulationselektroden ausgegeben.

### Quantifizierung der Stimulationsimpulse

Jeder ausgegebene Stimulationsimpuls wird gezählt und über einen Betrachtungszeitraum von z.B. 24 h als mittlere Stimulationsfrequenz verrechnet. Die mittlere Stimulationsfrequenz ist, individuell bei jedem Patienten zu bestimmen, und darf einen Maximalwert von etwa 0,2 Hz bis 2 Hz, insbesondere 0,7 bis 1 Hz, nicht überschreiten, um eine Überbeanspruchung des Muskels und eine mittelfristige Muskelzerstörung zu vermeiden.

### Ausgabe der mittleren Stimulationsfrequenz

Als wichtiger und neuer Feed-back-Mechanismus für den Träger des Muskelschrittmachers und für den behandelnden Arzt stellt die Ausgabe der mittleren Stimulationsfrequenz ein geeignetes Mittel dar, um muskuläre Überbelastungen frühzeitig zu erkennen, gegenzusteuern und so einer potentiellen Muskelgewebezerstörung vorzubeugen. In regelmäßigen, vom Arzt einstellbaren, Abständen übermittelt hierfür der Mikrocontroller per Datenfunk die mittlere Stimulationsfrequenz, die von einem tragbaren Patientenmonitor empfangen und für den Patienten wahrnehmbar, insbesondere sichtbar dargestellt wird. Sollte sich die mittlere Stimulationsfrequenz in einem kritischen Bereich befinden, sendet der Mikrocontroller die Daten unmittelbar; am Patientenmonitor wird dann eine Überlastungs-Warnmeldung ausgegeben. Der Patient kann durch Reduktion seiner körperlichen Tätigkeit seine Herzfrequenz und die Häufigkeit der Herzunterstützung des Muskels verringern.

### Impulseinsparmodus

Liegt die mittlere Stimulationsfrequenz in einem Bereich nahe dem oberen Grenzwert, so kann der automatische Impulseinsparungsmodus arbeiten, wenn er vom Arzt und/oder vom Träger des Implantats aktiviert wurde. In diesem Modus werden bei niedriger Aktivität die Stimulationsimpulse innerhalb des Stimulations-Burst so verteilt, dass initial eine ausreichende Impulsanzahl für eine Muskelkontraktion generiert wird, aber im weiteren Verlauf der Stimulation ein bis zwei Impulse durch Streckung, d.h. Reduzierung der Stimulationsfrequenz eingespart werden. Dies verringert die Anzahl der applizierten Impulse und somit die mittlere Stimulationsfrequenz. In Phasen hoher Aktivität arbeitet der Mechanismus aus Sicherheitsgründen nicht.

### Bereich 2:

### Patientenüberwachungs- und Kommunikationseinheit

Der zweite Bereich des Muskelschrittmachers dient der Überwachung des Patienten, der Erfassung und Speicherung der Messdaten und der telemetrischen Kommunikation mit der Umgebung.

### Echtzeit-Patientenüberwachung

Die Echtzeit-Patientenüberwachung erlaubt dem behandelnden Arzt, sich einen Überblick über die momentanen physiologischen Daten wie EKG, EMG und Blutdruck zu verschaffen. Hierzu werden nach Aktivierung des Messmoduls die Daten von der implantierten Vorrichtung über die jeweiligen Elektroden (EKG und Herzrhythmus; Herz-Sensing-Elektrode; EMG: Stimulationselektroden) und Sensoren (Absolutdrucksensor) erfasst, digitalisiert und komprimiert über eine Funkverbindung nach außen gebracht. An der empfangenden Monitoreinheit (Patientenmonitor) werden die Daten graphisch dargestellt und protokolliert. Eine Anschlussmöglichkeit des Patientenmonitors an einen Telekommunikationsweg, wie z.B. eine Telefonleitung gestattet ein ferndiagnostisches Monitoring des Patienten.

### Erfassung und Speicherung von "Langzeit"-physiologischen Daten

In zyklischen Abständen (einstellbar von 1 min bis 1 h) erfasst die Monitoreinheit des Muskelschrittmachers die Herzfrequenz und den systolischen sowie diastolischen Blutdruck. Diese Daten werden schrittmacherintern in einer Tabelle gespeichert und es wird eine Trendanalyse durchgeführt. Im Routinefall werden die gespeicherten Tabellenwerte einmal täglich zum Monitoreinheit übertragen, der diese dem behandelnden Arzt z.B. über eine Telefonwählverbindung übermittelt. Sollte die Trendanalyse jedoch eine lebensbedrohliche Gefährdung des Patienten ergeben, so wird das Ergebnis der Analyse unmittelbar telemetrisch zur Monitoreinheit übertragen. Bei Überschreitung von vom Arzt definierten Grenzwerten informiert die Monitoreinheit den behandelnden Arzt oder eine Notfallzentrale und übermittelt insbesondere über eine drahtlose Funkverbindung (UMTS/GSM) sowohl die Patientendaten als auch die GPS-Position des Patienten.

### Programmierbarkeit des Muskelschrittmachers

Zwecks Änderung des bestehenden Stimulationsmusters und zur Aktivierung/Deaktivierung der verschiedenen Betriebsmodi muss das implantierte Gerät auf äußere Anfragen reagieren können. Aus diesem Grund befindet sich der Telemetriebaustein und der Mikrocontroller 2 in zyklischen Abständen (einstellbar von Sekunden bis mehrere Stunden) in Empfangsstellung.

Folgende Parameter des Stimulationsmusters sind umprogrammierbar:
- Stimulationsspannung,
- Stimulationsphase,
- zeitliche Verteilung der Stimulationsimpulse,
- Art und Häufigkeit der Unterstützungsmodi,
- Verzögerungszeit zur R-Zacke,
- Dauer des Tag- und Nachtrhythmus bzw. des Arbeits- und Ruherhythmus,
- Elektrodenlage.

Folgende Betriebsmodi können aktiviert bzw. deaktiviert werden:
- Tag- und Nachtrhythmus bzw. Arbeits- und Ruhe-Rhythmus
- Impulseinsparmodus
- Echtzeit-Messdatenerfassung,
- Schrittmacherdiagnose-Programm,
- Impedanz-Messung der Stimulationselektroden
- Batteriespannungsmessung

Der Patientenmonitor (Monitoreinheit) ist ein batteriebetriebenes, vom Patienten tragbares System.

Es dient
- der Selbstkontrolle des Patienten, um die mittlere Stimulationsfrequenz im Bereich von 0,2 Hz bis 2 Hz, insbesondere 0,7 bis 1 Hz, pro z.B. 24 h zu erhalten, wobei eine farbig markierte Leuchtmittelanzeige (grün, gelb, rot) die, in zyklischen Abständen aktualisierte, mittlere Stimulationsfrequenz signalisiert;
- dem Telemonitoring des Patienten durch den behandelnden Arzt, wobei die vom Implantat erfassten physiologischen Echtzeit- und Langzeitdaten telemetrisch empfangen, protokolliert und weitervermittelt werden. Die Weitervermittlung der Daten erfolgt entweder über das integrierte Telefonmodem oder mittels Datenfunk in einem Funknetz, z.B. im UMTS/GSM-Netz;
- der Positionsbestimmung des Patienten in Notfallsituationen durch einen integrierten GPS-Empfänger;
- als Programmiergerät für grundlegende, vom Patienten selber einstellbare Stimulationsparameter und Betriebsmodi.

Der Patientenmonitor umfasst einen Mikrocontroller, ein Telemetrie-Modul, einen Standard-GPS-Empfänger, einen Standard-Modem-Baustein sowie ein UMTS/GSM-Modul.

Für die Visualisierung der mittleren Stimulationsfrequenz und für Statusmeldungen des Systems ist der Patientenmonitor mit einem grafischen Display sowie Leuchtmittel, insbesondere einer Leuchtdiodenanzeige ausgestattet. Die Dateneingabe erfolgt menügesteuert mittels Tastern. Alternativ ist eine Stifteingabe möglich.

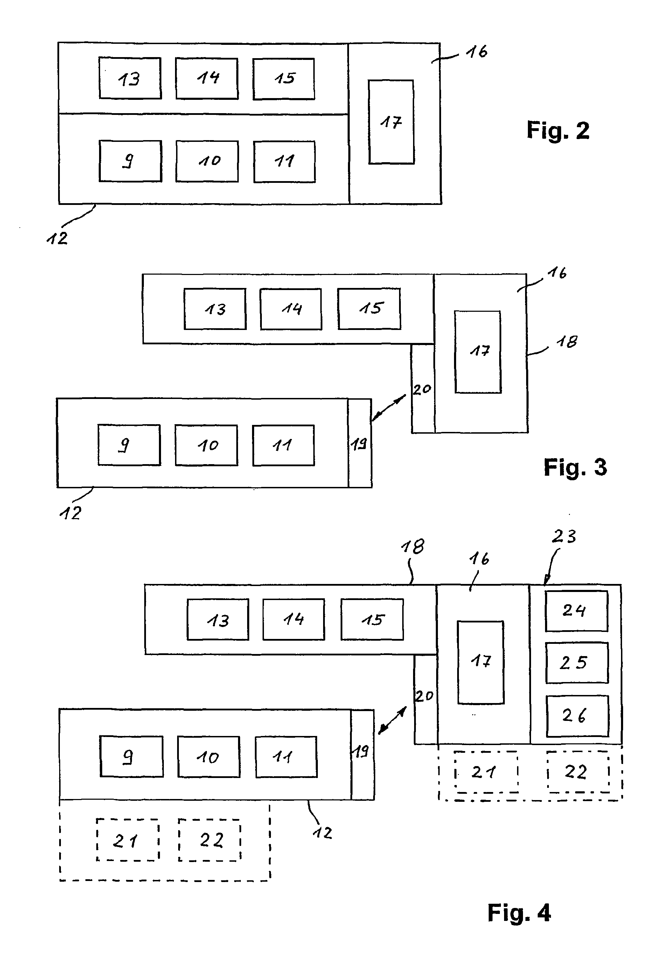

Die Figur 2 zeigt eine Vorrichtung zur Muskelstimulation mit einer Impulsgeneratoreinheit 9 zur Erzeugung und zum Aussenden eines elektrischen Stimulationsimpulses, eine Steuereinheit 10 zum Steuern der Impulsgeneratoreinheit 9, zum Einstellen von Amplitude und Frequenz der Stimulationsimpulse und zum Veranlassen, dass Stimulationsimpulse an einen zu stimulierenden Muskel abgegeben werden. Des Weiteren ist eine Erfassungseinheit 11 zur Erfassung des momentanen, spontanen oder stimulierten Herzrhythmus des Trägers der Vorrichtung vorgesehen. Diese Grundkomponenten der erfindungsgemäßen Vorrichtung zur Muskelstimulation sind in einem gemeinsamen Gehäuse 12 aufgenommen. Zusätzlich befinden sich in dem Gehäuse eine Zähleinheit 13 und eine Speichereinheit 14 zum Zählen und Speichern der Anzahl der innerhalb eines definierbaren Zeitraums abgegebenen Stimulationsimpulse. Die des Weiteren vorgesehene Bestimmungseinheit 15 dient zur Bestimmung einer mittleren Stimulationsfrequenz innerhalb eines definierbaren Zeitraums. Schließlich ist in dem Gehäuse 12 noch ein Impulseinsparmittel 16 mit einer Berechnungseinheit 17 vorgesehen. Die Berechnungseinheit 17 dient zur Berechnung eines Stimulationsmusters nach einer Gleichung, die in Abhängigkeit von der mittleren Stimulationsfrequenz das Stimulationsmuster bestimmt.

Die Ausführungsform der Figur 3 unterscheidet sich von derjenigen der Figur 2 dadurch, dass zwei räumlich getrennte Gehäuse 12, 18 vorgesehen sind. Das Gehäuse 12 mit der Impulsgeneratoreinheit 9, der Steuereinheit 10 und der Erfassungseinheit 11 einschließlich einer Sende- und Empfangseinheit 19 zur Kommunikation mit den Komponenten im anderen Gehäuse 18 ist zur Implantation in dem Körper eines Patienten vorgesehen. Die Zähleinheit 13, die Speichereinheit 14, die Bestimmungseinheit 15 sowie die Impulseinsparmittel 16 sind in diesem Ausführungsbeispiel räumlich getrennt von dem implantierten Gehäuse 12 angeordnet. Die Kommunikation erfolgt drahtlos mittels der Sende- und Empfangseinheit 19 und der Übertragungseinrichtung 20, die den jeweiligen Gehäusen 12, 18 zugeordnet sind.

Die Ausführungsform der Figur 4 ist hinsichtlich des extrakorporal angeordneten Gehäuses 18 um weitere Bestandteile ergänzt. In dem Gehäuse 18 ist zusätzlich ein Speichermodul 21 zur Speicherung des zeitlichen Verlaufs der Anzahl der abgegebenen Stimulationsimpulse vorgesehen, sowie eine Analyseeinheit 22 zur Ermittlung, wie oft und wann bestimmte Grenzwerte einer Herzfrequenz und/oder der mittleren Stimulationsfrequenz über- oder unterschritten wurden. Diese Komponenten sind mit strichpunktierter Linie eingezeichnet und können optional auch unmittelbar in dem implantierten Gehäuse 12 vorgesehen sein. In der Darstellung der Figur 4 sind sie daher mit unterbrochener Linie an das Gehäuse 12 angrenzend eingezeichnet. Da wesentliche Funktionskomponenten der erfindungsgemäßen Vorrichtung in der Ausführungsform der Figur 4 in dem Gehäuse 18 extrakorporal angeordnet sind, kann diese Baugruppe als Monitoreinheit 23 dienen. An der Monitoreinheit 23 ist eine Programmiereinrichtung 24 vorgesehen, wobei programmierte Steuerbefehle über die Übertragungseinrichtung 20 und die Sende- und Empfangseinheit 19 der Steuereinheit 10 in dem Gehäuse 12 zugeführt werden. In diesem Ausführungsbeispiel umfasst die Monitoreinheit 23 des weiteren Mittel 25 zum Senden und Empfangen von Positionsdaten sowie Mittel 26 zum senden und empfangen von Funksignalen zur Übertragung von patientenphysiologischen Daten an eine Anzeige- und Auswerteeinheit eines Empfängers. Die Monitoreinheit 23 besitzt in nicht näher dargestellter Weise optische und/oder akustische und/oder haptische Anzeigemittel zur Darstellung der mittleren Stimulationsfrequenz.

### Bezugszeichenaufstellung:

- 1 -: Mikrocontroller
- 2 -: Mikrocontroller
- 3 -: A/D-Wandler
- 4 -: D/A-Wandler
- 5 -: Telemetrieeinheit
- 6 -: Drucklogik
- 7 -: Serielle Schnittstelle
- 8 -: R-Zacken-Einheit
- 9 -: Impulsgeneratoreinheit
- 10 -: Steuereinheit
- 11 -: Erfassungseinheit
- 12 -: Gehäuse
- 13 -: Zähleinheit
- 14 -: Speichereinheit
- 15 -: Bestimmungseinheit
- 16 -: Impulseinsparmittel
- 17 -: Berechnungseinheit
- 18 -: Gehäuse
- 19 -: Sende- und Empfangseinheit
- 20 -: Übertragungseinrichtung
- 21 -: Speichermodul
- 22 -: Analyseeinheit
- 23 -: Monitoreinheit
- 24 -: Programmiereinheit
- 25 -: Mittel zum Senden und Empfangen von Positionsdaten
- 26 -: Mittel zum Senden und Empfangen von Funksignalen

- D -: Druckdaten
- P -: serielle Hochgeschwindigkeitsverbindung
- R -: Trigger-Signal
- S -: Stimulationsimpulse

## Patentansprüche

1. Vorrichtung zur Anregung von Muskelkontraktionen eines muskulär angetriebenen Herzunterstützungssystems, das parallel oder seriell zu einem erkrankten Herzen arbeitet, umfassend:
eine Impulsgeneratoreinheit (9) zur Erzeugung und zum Aussenden eines elektrischen Stimulationsimpulses;
eine Steuereinheit (10) zum Steuern der Impulsgeneratoreinheit (9) zum Einstellen von Amplitude und Frequenz der Stimulationsimpulse und zum Veranlassen, dass Stimulationsimpulse an einen zu stimulierenden Muskel abgegeben werden;
eine Erfassungseinheit (11) zur Erfassung des momentanen, spontanen oder stimulierten Herzrhythmus des Trägers der Vorrichtung;
ein Gehäuse (12), in welchem die Impulsgeneratoreinheit (9), die Steuereinheit (10) und die Erfassungseinheit (11) aufgenommen sind,
**dadurch gekennzeichnet, dass**
ein Speichermodul (21) zur Speicherung des zeitlichen Verlaufs der Anzahl der abgegebenen Stimulationsimpulse innerhalb eines definierbaren Zeitraums vorgesehen ist sowie
eine Zähleinheit (13) und eine Speichereinheit (14) zum Zählen und zur Speicherung der Anzahl der innerhalb eines definierbaren Zeitraumes abgegebenen Stimulationsimpulse vorgesehen sind, wobei die Stimulationsimpulse zu veränderbaren Stimulationsbursts gruppiert sind, wobei
eine Bestimmungseinheit (15) zur Bestimmung einer arithmetisch gemittelten, d.h. mittleren Stimulationsfrequenz innerhalb des definierbaren Zeitraums vorgesehen ist, wobei die mittlere Stimulationsfrequenz der Quotient aus der von der Speichereinheit (14) gespeicherten, innerhalb des definierbaren Zeitraums abgegebenen Stimulationsimpulse der veränderbaren Stimulationsbursts und dem definierten Zeitraum, in dem die Stimulationsimpulse gezählt und gespeichert worden sind, ist,
wobei eine kontinuierlich arbeitende Bewertungseinheit, zur Einhaltung von Grenzwerten für die mittlere Stimulationsfrequenz vorgesehen ist,
wobei Impulseinsparmittel (16) zur Reduzierung der mittleren Stimulationsfrequenz in Abhängigkeit von der von der Bewertungseinheit vorgegebenen maximalen mittleren Stimulationsfrequenz vorgesehen sind,
wobei die Impulseinsparmittel (16) eine Berechnungseinheit (17) zur Berechnung eines Stimulationsmusters nach einer Gleichung, die in Abhängigkeit von der mittleren Stimulationsfrequenz das Stimulationsmuster bestimmt, umfassen, wobei die Anzahl der Stimulationsimpulse je Stimulationsburst zur Reduzierung der mittleren Stimulationsfrequenz variierbar ist,
wobei eine vom Patienten extrakorporal getragene Monitoreinheit (23) zur Darstellung der mittleren Stimulationsfrequenz und Selbstkontrolle des Patienten vorgesehen ist
und wobei die Grenzwerte für die mittlere Stimutationsfrequenz in einem Bereich von 0,2 Stimulationsimpulsen pro Sekunde bis 2 Stimulationsimpulsen pro Sekunde individuell festlegbar sind.

2. Vorrichtung nach Anspruch 1, umfassend Mittel zur programmgesteuerten Übertragung der mittleren Stimulationsfrequenz von der Bestimmungseinheit zur Berechnungseinheit.

3. Vorrichtung nach Anspruch 1 oder 2, umfassend eine Analyseeinheit (22) zur Ermittlung, wie oft und wann bestimmte Grenzwerte der Herzfrequenz und/oder der mittleren Stimulationsfrequenz über- oder unterschritten wurden.

4. Vorrichtung nach einem der Ansprüche 1 bis 3, **dadurch gekennzeichnet, dass** die Zähleinheit (13) und die Speichereinheit (14) in dem Gehäuse (12) aufgenommen sind.

5. Vorrichtung nach Anspruch 4, **dadurch gekennzeichnet, dass** die Bestimmungseinheit (15) und/oder die Impulseinsparmittel (16) in das die Steuereinheit (10) aufnehmende Gehäuse (12) integriert sind.

6. Vorrichtung nach Anspruch 5, **dadurch gekennzeichnet, dass** das Speichermodul (21) und/oder die Analyseeinheit (22) in das die Steuereinheit (10) aufnehmende Gehäuse (12) integriert sind.

7. Vorrichtung nach einem der Ansprüche 1 bis 6, **dadurch gekennzeichnet, dass** die Monitoreinheit (23) eine Programmiereinrichtung (24) zur Erzeugung eines Programmiersignals und eine Übertragungseinrichtung zur Übertragung des Programmiersignals an eine Sende- und Empfangseinheit (19) in dem die Steuereinheit (10) aufnehmenden Gehäuse (12) aufweist.

8. Vorrichtung nach einem der Ansprüche 1 bis 7, **dadurch gekennzeichnet, dass** die Zähleinheit (13) und/oder die Speichereinheit (14) und/oder die Bestimmungseinheit (15) und/oder die Impulseinsparmittel (16) und/oder das Speichermodul (21) und/oder die Analyseeinheit (22) Bestandteil einer stationären und/oder vom Träger der Vorrichtung extrakorporal getragenen Monitoreinheit (23) sind.

9. Vorrichtung nach einem der Ansprüche 1 bis 8, wobei die mittlere Stimulationsfrequenz und/oder ein Größenbereich, in welchen die mittlere Stimulationsfrequenz fällt, an der Monitoreinheit (23) durch Anzeigemittel optisch und/oder akustisch und/oder haptisch darstellbar ist.

10. Vorrichtung nach einem der Ansprüche 1 bis 9, **dadurch gekennzeichnet, dass** die Monitoreinheit (23) Mittel (25) zum Senden und Empfangen von Positionsdaten umfasst.

11. Vorrichtung nach einem der Ansprüche 1 bis 10, **dadurch gekennzeichnet, dass** die Monitoreinheit (23) Mittel (26) zum Senden und Empfangen von Funksignalen zur Übertragung von patienten-physiologischen Daten an eine Anzeige- und Auswerteeinheit eines Empfängers umfasst.

12. Vorrichtung nach einem der Ansprüche 1 bis 11, **dadurch gekennzeichnet, dass** von der Impulsgeneratoreinheit (9) biphasische Stimulationsimpulse abgebbar sind.

13. Vorrichtung nach einem der Ansprüche 1 bis 12, **dadurch gekennzeichnet, dass** ein Energiespeicher im Gehäuse (12) transkutan aufladbar ist.

14. Vorrichtung nach einem der Ansprüche 1 bis 13, **dadurch gekennzeichnet, dass** der definierbare Zeitraum mindestens 30 Minuten umfasst.

15. Vorrichtung nach einem der Ansprüche 1 bis 14, **dadurch gekennzeichnet, dass** der definierbare Zeitraum mindestens 12 Stunden umfasst.

16. Vorrichtung nach einem der Ansprüche 1 bis 15, dass der definierbare Zeitraum 24 Stunden umfasst.

17. Vorrichtung nach einem der Ansprüche 1 bis 16, **dadurch gekennzeichnet, dass** die Amplituden der Stimulationsimpulse innerhalb eines Stimulationsbursts variierbar sind.

18. Vorrichtung nach einem der Ansprüche 1 bis 17, **dadurch gekennzeichnet, dass** die Impulsbreiten der Stimulationsimpulse inner halb eines Stimulationsbursts variierbar sind.

19. Vorrichtung nach einem der Ansprüche 1 bis 18, **dadurch gekennzeichnet, dass** der Interimpulsabstand zwischen zwei Stimutationsimpulsen innerhalb eines Stimulationsbursts variierbar ist.

## Claims

1. A device to induce muscle contractions of a muscularly driven heart support system that operates parallel to or in series with a diseased heart, comprising:
• a pulse generating unit (9) to produce and emit an electrical stimulation pulse;
• a control unit (10) to control the pulse generating unit (9) to adjust the amplitude and frequency of the stimulation pulse and to ensure that the stimulation pulses are delivered to a muscle to be stimulated;
• a detection unit (11) to detect the momentary, spontaneous or stimulated heart rhythm of the bearer of the device;
• a housing (12), in which the pulse generator unit (9), the control unit (10) and the detection unit (11) are accommodated;
**characterised in that**
• a memory module (21) is provided to store the temporal progress of the number of stimulation pulses delivered within a definable period of time, as well as
• a counting unit (13) and a memory unit (14) to count and store the number of stimulation pulses delivered within a definable period are provided, while the stimulation pulses are grouped into modifiable stimulation bursts, wherein
• a determination unit (15) is provided to determine an arithmetically averaged, i.e. average, stimulation frequency within the definable period of time, while the average stimulation frequency is the quotient of the stimulation pulses of the modifiable stimulation bursts stored by the memory unit (14) and delivered within the defined period of time and of the defined period of time, in which the stimulation pulses were counted and stored,
• wherein a continuously operating evaluation unit is provided to adhere to the limit values for the average stimulation frequency,
• wherein pulse saving means (16) are provided to reduce the average stimulation frequency depending on the maximum average stimulation frequency determined by the evaluation unit,
• wherein the pulse saving means (16) comprise a calculating unit (17) to calculate a stimulation pattern according to an equation, that determines the stimulation pattern depending on the average stimulation frequency, while the number of stimulation pulses per stimulation burst is variable to reduce the average stimulation frequency,
• wherein a monitoring unit (23), carried extracorporeally by the patient, is provided to illustrate the average stimulation frequency and for self-control of the patient, and
• wherein the limit values for the average stimulation frequency are individually adjustable in a range of 0.2 stimulation pulses/sec up to 2 stimulation pulses/sec.

2. A device according to claim 1, comprising means for the program-controlled transmission of the average stimulation frequency from the determination unit to the calculating unit.

3. A device according to claim 1 or 2, comprising an analysis unit (22) to determine as to how often and when established limit values of the heart rate and/or of the average stimulation frequency are exceeded or not reached.

4. A device according to any one of claims 1 to 3, **characterised in that** the counting unit (13) and the memory unit (14) are accommodated in the housing (12).

5. A device according to claim 4, **characterised in that** the determination unit (15) and/or the pulse saving means (16) are integrated in the housing (12) that accommodates the control unit (10).

6. A device according to claim 5, **characterised in that** the memory module (21) and/or the analysing unit (22) are integrated in the housing (12) that accommodates the control unit (10).

7. A device according to any one of claims 1 to 6, **characterised in that** the monitoring unit (23) has a programming device (24) to produce a programming signal and a transfer device to transfer the programming signal to a transmission and reception unit (19) in the housing (12), accommodating the control unit (10).

8. A device according to any one of claims 1 to 7, **characterised in that** the counting unit (13) and/or the memory unit (14) and/or the determination unit (15) and/or the pulse saving means (16) and/or the memory module (21) and/or the analysing unit (22) are components of a monitoring unit (23) that is stationary and/or is extracorporeally carried by the bearer.

9. A device according to any one of claims 1 to 8, wherein the average stimulation frequency and/or a magnitude range, into which the average stimulation frequency falls, can be illustrated on the monitoring unit (23) optically and/or acoustically and/or in a tactile manner via indicating means.

10. A device according to any one of claims 1 to 9, **characterised in that** the monitoring unit (23) comprises means (25) to transmit and receive positional data.

11. A device according to any one of claims 1 to 10, **characterised in that** the monitoring unit (23) comprises means (26) to transmit and receive wireless signals to transmit physiological data of the patient to a display and evaluating unit of a receiver.

12. A device according to any one of claims 1 to 11, **characterised in that** biphased stimulation pulses can be emitted by the pulse generating unit (9).

13. A device according to any one of claims 1 to 12, **characterised in that** an energy storage in the housing (12) can be transcutaneously charged.

14. A device according to any one of claims 1 to 13, **characterised in that** the definable period of time is at least 30 minutes.

15. A device according to any one of claims 1 to 14, **characterised in that** the definable period of time is at least 12 hours.

16. A device according to any one of claims 1 to 15, **characterised in that** the definable period of time is at least 24 hours.

17. A device according to any one of claims 1 to 16, **characterised in that** the amplitudes of the stimulation pulses are variable within a simulation burst.

18. A device according to any one of claims 1 to 17, **characterised in that** the pulse widths of the stimulation pulses are variable within a stimulation burst.

19. A device according to any one of claims 1 to 18, **characterised in that** the pulse interval between two stimulation pulses are variable within a stimulation burst.

## Revendications

1. Dispositif pour stimuler les contractions musculaires d'un système d'assistance cardiaque commandé musculairement, lequel fonctionne parallèlement ou en série par rapport à un coeur malade, comprenant :
- une unité génératrice d'impulsions (9) destinée à générer et à émettre une impulsion électrique de stimulation ;
- une unité de commande (10) destinée à commander l'unité génératrice d'impulsions (9) afin de régler l'amplitude et la fréquence des impulsions de stimulation et de faire en sorte que les impulsions de stimulation soient transmises à un muscle à stimuler ;
- une unité de détection (11) pour détecter le rythme cardiaque momentané, spontané ou stimulé du porteur du dispositif ;
- un boîtier (12) dans lequel sont logées l'unité génératrice d'impulsions (9), l'unité de commande (10) et l'unité de détection (11), **caractérisé en ce qu'**est prévu
- un module de mémoire (21) destiné à mettre en mémoire le tracé temporel du nombre d'impulsions de stimulation transmises en l'espace d'une période définissable, et **en ce que** sont prévues
- une unité de comptage (13) et une unité de mémoire (14) pour compter et mémoriser le nombre d'impulsions de stimulation transmises en l'espace d'une période définissable, les impulsions de stimulation étant groupées en rafales modifiables de stimulation, étant précisé qu'il est prévu
- une unité de détermination (15) destinée à déterminer une fréquence de stimulation dont la moyenne a été calculée de façon arithmétique, c'est-à-dire une fréquence moyenne de stimulation en l'espace de la période définissable, la fréquence de stimulation étant le quotient des impulsions de stimulation des rafales modifiables de stimulation transmises en l'espace d'une période définissable et mémorisées par l'unité de mémoire (14) et la période définissable, dans laquelle les impulsions de stimulation ont été comptées et mémorisées,
- une unité d'évaluation fonctionnant en continu étant prévue pour respecter les valeurs limites pour la fréquence moyenne de stimulation,
- des économiseurs d'impulsions (16) étant prévus pour réduire la fréquence moyenne de stimulation en fonction de la fréquence moyenne maximale de stimulation prédéterminée par l'unité d'évaluation,
- les économiseurs d'impulsions (16) comprenant une unité de calcul (17) destinée à calculer un modèle de stimulation d'après une équation qui détermine le modèle de stimulation en fonction de la fréquence moyenne de stimulation, sachant que le nombre d'impulsions de stimulation par rafale de stimulation est modulable afin de réduire la fréquence moyenne de stimulation,
- une unité de contrôle (23) portée de façon extracorporelle par le patient étant prévue pour représenter la fréquence moyenne de stimulation et l'autocontrôle du patient
- et les valeurs limites pour la fréquence moyenne de stimulation pouvant être fixées individuellement dans une plage de 0,2 impulsion de stimulation par seconde jusqu'à 2 impulsions de stimulation par seconde.

2. Dispositif selon la revendication 1, comprenant des moyens pour la transmission commandée par programme de la fréquence moyenne de stimulation depuis l'unité de détermination jusqu'à l'unité de calcul.

3. Dispositif selon la revendication 1 ou 2, comprenant une unité d'analyse (22) destinée à déterminer combien de fois et à quel moment certaines valeurs limites de la fréquence cardiaque et/ou de la fréquence moyenne de stimulation ont été dépassées vers le haut ou vers le bas.

4. Dispositif selon l'une quelconque des revendications 1 à 3, **caractérisé en ce que** l'unité de comptage (13) et l'unité de mémoire (14) sont logées dans le boîtier (12).

5. Dispositif selon la revendication 4, **caractérisé en ce que** l'unité de détermination (15) et/ou les économiseurs d'impulsions (16) sont intégrés dans le boîtier (12) recevant l'unité de commande (10).

6. Dispositif selon la revendication 5, **caractérisé en ce que** le module de mémoire (21) et/ou l'unité d'analyse (22) sont intégrés dans le boîtier (12) recevant l'unité de commande (10).

7. Dispositif selon l'une quelconque des revendications 1 à 6, **caractérisé en ce que** l'unité de contrôle (23) comporte un dispositif de programmation (24) pour générer un signal de programmation et un dispositif de transmission pour transmettre le signal de programmation à une unité d'émission et de réception (19) dans le boîtier (12) recevant l'unité de commande (10).

8. Dispositif selon l'une des revendications 1 à 7, **caractérisé en ce que** l'unité de comptage (13) et/ou l'unité de mémoire (14) et/ou l'unité de détermination (15) et/ou les économiseurs d'impulsions (16) et/ou le module de mémoire (21) et/ou l'unité d'analyse (22) font partie d'une unité de contrôle (23) stationnaire et/ou portée de façon extracorporelle par le porteur du dispositif.

9. Dispositif selon l'une des revendications 1 à 8, la fréquence moyenne de stimulation et/ou un ordre de grandeur, dans lequel se situe la fréquence moyenne de stimulation, peut être représenté de façon optique et/ou acoustique et/ou haptique sur l'unité de contrôle (23) par des moyens d'affichage.

10. Dispositif selon l'une quelconque des revendications 1 à 9, **caractérisé en ce que** l'unité de contrôle (23) comprend des moyens (25) pour émettre et recevoir des données de position.

11. Dispositif selon l'une quelconque des revendications 1 à 10, **caractérisé en ce que** l'unité de contrôle (23) comporte des moyens (26) pour émettre et recevoir des signaux radio en vue de transmettre des données physiologiques du patient à une unité d'affichage et d'interprétation d'un récepteur.

12. Dispositif selon l'une quelconque des revendications 1 à 11, **caractérisé en ce que** l'unité génératrice d'impulsions (9) peut transmettre des impulsions de stimulation à deux phases.

13. Dispositif selon l'une quelconque des revendications 1 à 12, **caractérisé en ce qu'**un accumulateur d'énergie peut être chargé dans le boîtier (12) par voie transcutanée.

14. Dispositif selon l'une quelconque des revendications 1 à 13, **caractérisé en ce que** la période définissable comprend au moins 30 minutes.

15. Dispositif selon l'une quelconque des revendications 1 à 14, **caractérisé en ce que** la période définissable comprend au moins 12 heures.

16. Dispositif selon l'une quelconque des revendications 1 à 15, **caractérisé en ce que** la période définissable comprend 24 heures.

17. Dispositif selon l'une quelconque des revendications 1 à 16, **caractérisé en ce que** les amplitudes des impulsions de stimulation sont modulables en l'espace d'une rafale de stimulation.

18. Dispositif selon l'une quelconque des revendications 1 à 17, **caractérisé en ce que** les largeurs d'impulsion des impulsions de stimulation sont modulables en l'espace d'une rafale de stimulation.

19. Dispositif selon l'une quelconque des revendications 1 à 18, **caractérisé en ce que** l'intervalle entre deux impulsions de stimulation est modulable en l'espace d'une rafale de stimulation.
